(19) Europäisches Patentamt
European Patent Office
Office européen des brevets

(11) Publication number: **0 385 271 B1**

(12) **EUROPEAN PATENT SPECIFICATION**

(45) Date of publication of patent specification: **25.10.95**

(51) Int. Cl.[6]: **C07D 215/36**, C07F 9/60

(21) Application number: **90103451.2**

(22) Date of filing: **22.02.90**

(54) **Tetrahydroquinolines and method of preparation.**

(30) Priority: **27.02.89 US 316277**

(43) Date of publication of application:
**05.09.90 Bulletin 90/36**

(45) Publication of the grant of the patent:
**25.10.95 Bulletin 95/43**

(84) Designated Contracting States:
**DE FR GB**

(56) References cited:
**GB-A- 2 069 498**

**CHEMICAL ABSTRACTS vol. 64, no.3,31 January 1966, column 3471a-c, Columbus, Ohio, US; L.S. POVAROV et al. :"Reactions of anils with vinyl butyl sulfide "& Izv. Akad. Nauk. SSSR, Ser. Khim. 1965,vol. 10, pages 1891-1893**

(73) Proprietor: **EASTMAN KODAK COMPANY**
**343 State Street**
**Rochester,**
**New York 14650-2201 (US)**

(72) Inventor: **Lau, Philip T.S., c/o Eastman Kodak Company**
**Patent Department,**
**343 State Street**
**Rochester,**
**New York 14650 (US)**

(74) Representative: **Brandes, Jürgen, Dr.rer.nat. et al**
**Schweigerstrasse 2**
**D-81541 München (DE)**

EP 0 385 271 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to a new process for the preparation of 1,2,3,4-tetrahydroquinolines and to compounds prepared by such a process.

In the synthesis of tetrahydroquinolines as in the preparation of other heterocyclic compounds, it has been highly desirable to form the heterocyclic ring at the same time as the introduction of a functional or functionalizable group on the heterocyclic ring without the need for a series of synthetic steps. This is desirable for the formation of heterocyclic compounds that are useful in, for example, the formation of photographically useful compounds without resorting to multistep procedures that involve harsh reaction conditions that may destroy the useful functional group on the heterocyclic ring. This is useful in, for example, the synthesis of dyes, photographic couplers and other compounds that are useful in photographic elements and processes.

Prior processes for preparation of tetrahydroquinolines involve first the formation and isolation of quinolines (Chem. Abstracts 62, 5252, 1965 and U.S. Patent 3,896,131) or the formation of a mixture of quinolines and tetrahydroquinolines (U.S. Patents 4,301,288 and 4,301,289) and then reduction of the quinolines or mixtures to the tetrahydroquinolines. The reaction times for these processes are long and the reaction temperatures are far above room temperature.

The present invention solves these problems by providing a process for preparing tetrahydroquinolines in one step under mild reaction conditions and short reaction times, that is, under ambient temperature and pressure conditions, preferably at a temperature within the range of 15 °C. to 30 °C., typically at room temperature and at atmospheric pressure. No heating of the reactants is required. The reaction is complete within 1 to 2 hours. The tetrahydroquinoline products are not contaminated with quinolines. This is made possible by the use of nucleophilic reagents in the reaction mixture which stop the reaction at the tetrahydroquinoline stage. No prior art make specific use of nucleophilic reagents. The nucleophilic reagents enable direct introduction of functional or functionalizable groups into the position of the heterocyclic ring in a one-step process.

The present invention provides a method of preparing a 1,2,3,4-tetrahydroquinoline containing an alkyl group in the 2- position and an alkyl, aryl or heterocyclic thio, sulfonyl or phosphonium group in the 4-position comprising the step of reacting (a) an unsubstituted or substituted aniline salt represented by the formula:

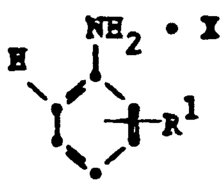

wherein $R^1$ is hydrogen or a substituent that does not adversely affect the reaction with other reactants, and X is a salt with

(b) a compound represented by the formulas: $R^3SQ$ and $Ar_3P$, wherein $R^3$ is an unsubstituted or substituted alkyl containing 1 to 20 carbon atoms, phenyl, tolyl and 4-chlorophenyl, phenyltetrazole and benzothiazole, Q is H or $O_2^{\ominus}$ ; and Ar is phenyl and tolyl and

(c) an enolizable alkyl aldehyde containing an alpha hydrogen and 2 to 15 carbon atoms in an organic solvent for the reactants.

The novel intermediates prepared by this one step process are useful for preparation of, for example, photographically useful compounds, such as photographically useful couplers for use in photographic elements and processes for formation of dye images.

In the formula X is particularly an acid salt, such as HCl or sulfate salt. The aniline reactant accordingly also includes the salts of such an aniline, such as the acid salts, for instance hydrochloride and sulfate salts.

Examples of useful aniline reactants (a) include aniline, 2-hydroxyaniline, 2-methyl-5-hydroxyaniline, and 2-alkoxyaniline, such as 2-methoxyaniline, 2-hexyloxyaniline and 2-alkylaniline, such as 2-methylaniline and 2-butylaniline, 4-chloroaniline, 4-cyanoaniline, 2-acetamido-4-chloroaniline, ethyl-2-aminobenzoate and 3,5-dimethylaniline.

The compounds of the formulas $R^3SQ$ and $Ar_3P$ are nucleophiles under acidic reaction conditions. These nucleophiles react only in the 4-position of the 1,2,3,4-tetrahydroquinoline ring. Useful alkyl groups,

which can be presented by $R^3$ are, for example, alkyl groups containing 1 to 20 carbon atoms, such as methyl, ethyl, propyl, n-butyl, t-butyl, octyl and eicosyl as well as such substituted alkyl groups as chloroethyl and hydroxyethyl.

Particularly useful reactants are arenethiols, arenesulfinic acids and triarylphosphines.

The alkyl group of the alkyl aldehyde reactant can be unsubstituted or substituted with groups that do not adversely affect the described synthesis. Useful alkyl groups include for example, methyl, ethyl, propyl, n-butyl, octyl and dodecyl. Particularly useful alkyl aldehyde reactants are acetaldehyde, propionaldehyde and dodecyl aldehyde.

The described reaction is carried out in a solvent for the reactants, preferably in a polar solvent in which the aniline salt is soluble. Examples of useful solvents are ethanol, methanol, acetonitrile and dimethylformamide (DMF).

Typically, an excess of the aniline and aldehyde over the nucleophilic reagent is useful to achieve optimum yield.

The conditions for the described synthesis are typically ambient conditions of temperature and pressure. The reaction is typically carried out at a temperature that is within the range of 15 °C. to 30 °C., such as at room temperature, that is about 20 °C. at atmospheric pressure. No external heating is required.

The reaction is preferably carried to completion in the presence of a strong acid, such as HCl or sulfuric acid, and requires no catalyst or other reaction promoting means.

A preferred method according to the invention comprises the method of preparing a 4-phenylthio or sulfonyl-1,2,3,4-tetrahydroquinoline represented by the formula:

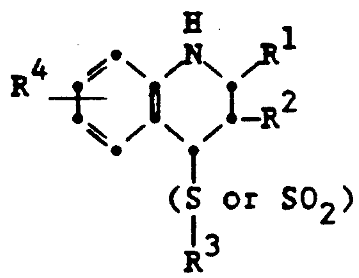

comprising the step of reacting an aniline salt represented by the formula:

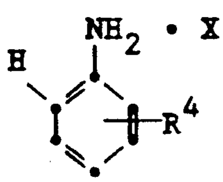

with a thiol or sodium sulfinate represented by the formula: $R^3$-SH or $R^3$-$SO_2Na$ and an alkyl aldehyde represented by the formula: $R^5CHO$ in an organic solvent for the reactants at a temperature within the range of 15 °C. to 30 °C., wherein

$R^1$ is alkyl containing 1 to 20 carbon atoms, typically methyl, ethyl, propyl or dodecyl;

$R^2$ is hydrogen or alkyl containing one carbon less than the alkyl group selected for $R^1$;

$R^3$ is an unsubstituted or substituted alkyl, containing 1 to 20 carbon atoms, phenyl, tolyl and 4-chlorophenyl,

$R^4$ is hydrogen or a substituent, that does not adversely affect the reaction with other reactants, such as -OH, $OCH_3$, $CH_3$, Cl, CN, -$CO_2$Et, -$NHCOCH_3$ or -$OC_{14}H_{29}$-n;

$R^5$ is an enolizable alkyl; and,

X is a salt, particularly an acid salt such as HCl or sulfate salt. Et herein is ethyl.

A particularly preferred method comprises the method of preparing 2-methyl-4-phenylthio-1,2,3,4-tetrahydroquinoline comprising the step of reacting aniline hydrochloride with acetaldehyde and thiophenol in ethanol at a temperature within the range of 15 °C. to 30 °C.

New 1,2,3,4-tetrahydroquinolines containing an alkyl group in the 2- position and an alkyl, aryl or heterocyclic thio, sulfone or phosphonium group in the 4- position can be prepared by the described

method of synthesis. These compounds can be useful for preparing antifungal, antimicrobial and antistain agents. They can also be useful for preparing photographically useful compounds, such as dye stabilizing agents, image couplers, image modifiers, development inhibitors and accelerators, image dyes, and masking dyes.

The following examples further illustrate the invention. In each of the following examples all the reactants were used as obtained from commercial sources or prepared without further purification. Thin layer chromotography (TLC) was performed by procedures accepted in the organic compound analysis art on glass plates precoated with silica gel containing fluorescent indicator. Nuclear magnetic resonance (NMR) and mass spectra for the compounds prepared were obtained on commercial instruments.

Example 1: Preparation of 2-methyl-4-phenylthio-1,2,3,4-tetrahydroquinoline hydrochloride

To a stirred solution of aniline hydrochloride (26.0 g., 0.2 mol) in 150 mL of ethanol was added acetaldehyde (8.8 g., 0.2 mol) over a period of 15 minutes while keeping the reaction temperature below 30 °C. by means of a water bath. After stirring for 10 minutes thiophenol (9.9 g, 0.09 mol) was added in one portion. The progress of the reaction was followed by TLC (5:1 methylene chloride/ethyl acetate). The reaction mixture was stirred at room temperature (20 °C.) for one hour during which time a white solid precipitated. The white solid was collected and washed with 20 mL of ether to provide 14.5 g of desired product. An additional 4.0 g of solid was isolated from the filtrate to provide a total yield of 18.5 g (70%) of TLC pure product having a melting point of 166 °-167 °C. The solid product was readily recrystallized from ethanol. The desired product was identified by elemental analysis and NMR spectra.

The following additional compounds were also prepared by means of the procedures described in Example 1:

## TABLE I

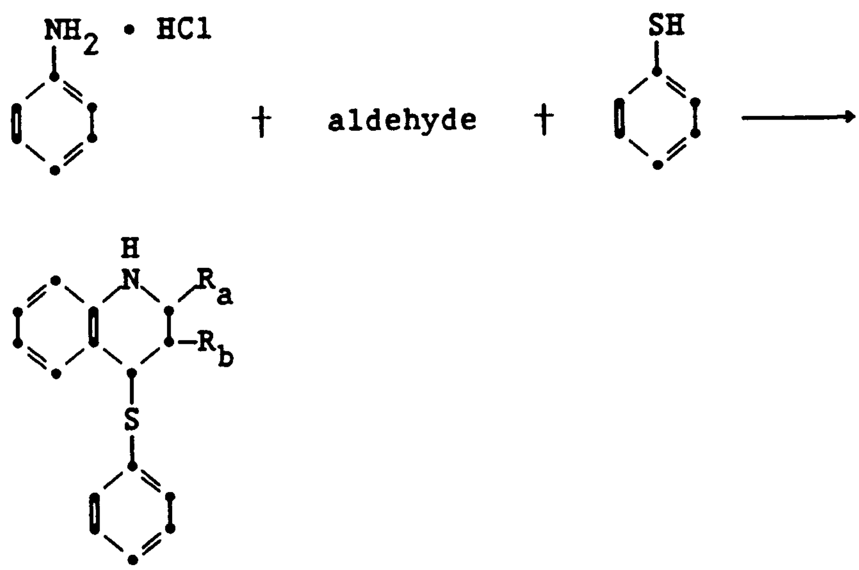


| Example No. | Aldehyde | $R_a$ | $R_b$ | Pct. Yield | Melting Point (°C.) |
|---|---|---|---|---|---|
| 2 | $CH_3CH{=}CHCHO$ | $CH_3$ | H | 59 | 166–167 (HCl salt) |
| 3 | $CH_3CH_2CHO$ | $CH_3CH_2$ | $CH_3$ | 71 | 164–166 (HCl salt) |
| 4 | $CH_3(CH_2)_2CHO$ | $CH_3(CH_2)_2$ | $CH_3CH_2$ | 80 | Oil |
| 5 | $CH_3(CH_2)_{10}CHO$ | $CH_3(CH_2)_{10}$ | $CH_3(CH_2)_9$ | 92 | Oil |

Example 6:

Using the procedure of Example 1, ethyl 2-aminobenzoate hydrochloride was reacted with acetaldehyde and thiophenol to form 2-methyl-4-phenylthio-8-ethoxycarbonyl-1,2,3,4-tetrahydroquinoline

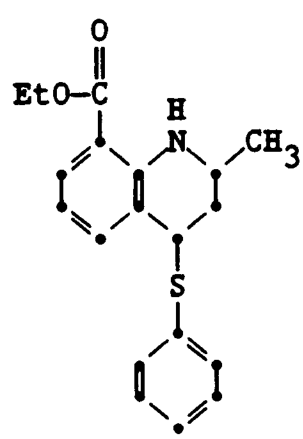

in 87% yield having a melting point of 57°-58°C.

Example 7: Preparation of 2-methyl-4-phenyl-sulfonyl-1,2,3,4-tetrahydroquinoline

To a solution of aniline hydrochloride (26 g., 0.20 mol) in 150 mL of ethanol was added with stirring acetaldehyde (8.8 g., 0.20 mol) dropwise over a period of 15 minutes. The temperature of the reaction mixture was kept below 30°C. by means of a water bath. The mixture was stirred for 10 minutes then a solution of sodium benzenesulfinate (14.8 g, 0.09 mol) dissolved in 30 mL water was added in one portion. Within 5 minutes after the addition a white solid product precipitated from the reaction mixture. After two hours of stirring at room temperature (20°C.), the solid was collected, washed with water and dried. The yield of TLC pure product was 24.1 g (85%) and the desired product had a melting point of 149°-150°C. The solid was recrystallized from ethanol. The desired product was also identified by elemental analysis and NMR spectra.

Example 8:

Using the procedure of Example 7, aniline hydrochloride was reacted with propionaldehyde and sodium benzenesulfinate to form 2-ethyl-3-methyl-4-phenylsulfonyl-1,2,3,4-tetrahydroquinoline in 83% yield having a melting point of 152°-153°C.

Example 9:

Using the procedure of Example 1, aniline hydrochloride was reacted with acetaldehyde and triphenyl-phosphine to form:

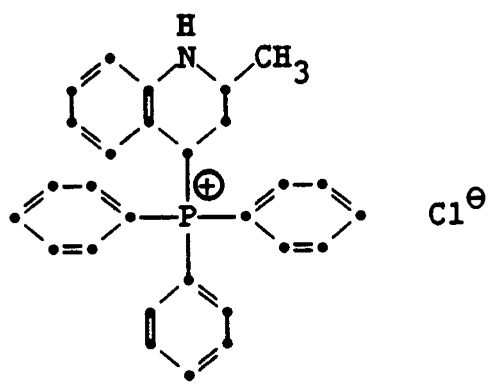

in 72% yield having a melting point of 166°-168°C. as the chloride salt.

**Claims**

**1.** A method of preparing a 1,2,3,4-tetrahydroquinoline containing an alkyl group in the 2- position and an alkyl, aryl or heterocyclic thio, sulfonyl or phosphonium group in the 4- position comprising the step of reacting (a) an unsubstituted or substituted aniline salt represented by the formula:

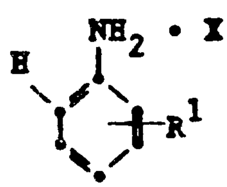

wherein $R^1$ is hydrogen or a substituent that does not adversely affect the reaction with other reactants,

and X is a salt with
(b) a compound represented by the formulas: $R^3SQ$ and $Ar_3P$, wherein $R^3$ is an unsubstituted or substituted alkyl containing 1 to 20 carbon atoms, phenyl, tolyl and 4-chlorophenyl, phenyltetrazole and benzothiazole,
Q is H or $O_2^{\ominus}$; and Ar is phenyl and tolyl and
(c) an enolizable alkyl aldehyde containing an alpha hydrogen and 2 to 15 carbon atoms in an organic solvent for the reactants.

**2.** A method as in claim 1, wherein the organic solvent is a polar organic solvent selected from the group consisting of ethanol, methanol, acetonitrile and dimethylformamide (DMF).

**3.** A method as in claims 1 - 2, wherein the reaction is carried out at a temperature within the range of 15 ° C. to 30 ° C.

**4.** A method as in claims 1 - 3 of preparing a 4-phenylthio or sulfonyl-1,2,3,4-tetrahydroquinoline represented by the formula:

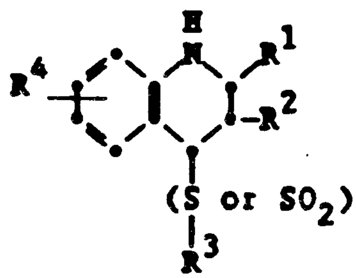

comprising the step of reacting an aniline salt represented by the formula:

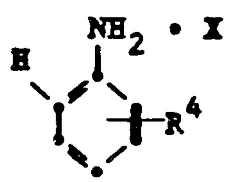

with a thiol or sulfinate represented by the formula: $R^3$-SH or $R^3$-$SO_2^{\ominus}$ and an alkyl aldehyde represented by the formula: $R^5CHO$ in on organic solvent for the reactants at a temperature within the range of 15 ° C. to 30 ° C., wherein
$R^1$ is alkyl containing 1 to 20 carbon atoms;
$R^2$ is hydrogen or alkyl containing one carbon less than the alkyl group selected for $R^1$;
$R^3$ is an unsubstituted or substituted alkyl containing 1 to 20 carbon atoms; phenyl, tolyl and 4-chlorophenyl, phenyltetrazole and benzothiazole;
$R^4$ is hydrogen or a substituent, that does not adversely affect the reaction with other reactants;
$R^5$ is an enolizable alkyl; and,
X is a salt.

## Patentansprüche

**1.** Verfahren zur Herstellung eines 1,2,3,4,-Tetrahydrochinolins mit einer Alkylgruppe in der 2-Position und einer Alkyl-, Aryl- oder heterocyclischen Thio-, Sulfonyl- oder Phosphoniumgruppe in der 4-Position mit der Stufe der Umsetzung von (a) einem unsubstituierten oder substituierten Anilinsalz, dargestellt durch die Formel:

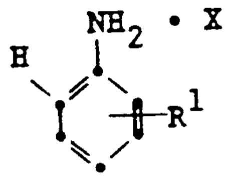

worin $R^1$ für Wasserstoff steht oder einen Substituenten, der die Umsetzung mit anderen Reaktionskomponenten nicht nachteilig beeinflußt, und worin X für ein Salz steht, mit
(b) einer Verbindung, die durch die Formeln: $R^3SQ$ und $Ar_3P$ dargestellt wird, worin $R^3$ steht für einen unsubstituierten oder substituierten Alkylrest mit 1 bis 20 Kohlenstoffatomen, Phenyl, Tolyl und 4-Chlorophenyl, Phenyltetrazol und Benzothiazol, worin Q steht für H oder $O_2^{\ominus}$ ; und worin Ar ein Phenyl- oder Tolylrest ist, und
(c) einem enolysierbaren Alkylaldehyd mit einem alpha-Wasserstoffatom und 2 bis 15 Kohlenstoffatomen in einem organischen Lösungsmittel für die Reaktionskomponenten.

**2.** Verfahren nach Anspruch 1, bei dem das organische Lösungsmittel ein polares organisches Lösungsmittel ist, das ausgewählt ist aus der Gruppe bestehend aus Ethanol, Methanol, Acetonitril und Dimethylformamid (DMF).

**3.** Verfahren nach Ansprüchen 1 - 2, bei dem die Umsetzung bei einer Temperatur im Bereich von 15 °C bis 30 °C durchgeführt wird.

**4.** Verfahren nach Ansprüchen 1 - 3, zur Herstellung eines 4-Phenylthio- oder Sulfonyl-1,2,3,4-tetrahydrochinolins gemäß der Formel

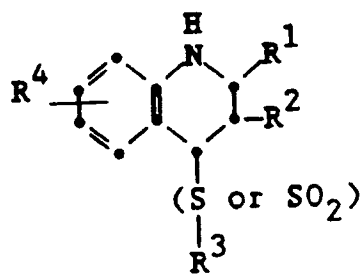

mit der Stufe der Umsetzung eines Anilinsalzes, dargestellt durch die Formel:

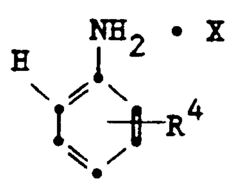

mit einem Thiol oder Sulfinat, dargestellt durch die Formel: $R^3$-SH oder $R^3$-$SO_2^{\ominus}$ und einem Alkylaldehyd, dargestellt durch die Formel: $R^5CHO$ in einem organischen Lösungsmittel für die Reaktionskomponenten bei einer Temperatur innerhalb des Bereiches von 15 °C bis 30 °C, wobei bedeuten:
- $R^1$ einen Alkylrest mit 1 bis 20 Kohlenstoffatomen;
- $R^2$ ein Wasserstoffatom oder einen Alkylrest mit einem Kohlenstoffatom weniger als in der Alkylgruppe, die für $R^1$ ausgewählt ist;
- $R^3$ eine unsubstituierte oder substituierte Alkylgruppe mit 1 bis 20 Kohlenstoffatomen; Phenyl,

Tolyl und 4-Chlorophenyl, Phenyltetrazol und Benzothiazol;
$R^4$ ein Wasserstoffatom oder ein Substituent, der die Reaktion mit anderen Reaktionskomponenten nicht nachteilig beeinflußt;
$R^5$ einen enolysierbaren Alkylrest; und
X ein Salz.

## Revendications

1. Procédé de préparation d'une 1,2,3,4-tétrahydroquinoéline contenant un groupe alkyle en position 2 et un groupe alkyl-, aryl- ou hétérocyclylthio, -sulfonyle ou -phosphonium en position 4, comprenant l'étape de réaction de (a) un sel d'aniline substituée ou non représenté par la formule :

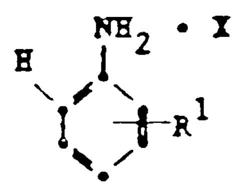

dans laquelle $R^1$ est l'hydrogène ou un substituant qui ne gêne pas la réaction avec les autres réactifs et X est un sel, avec

(b) un composé représenté par les formules $R^3SQ$ et $Ar_3P$, dans lesquelles $R^3$ est un groupe alkyle en $C_1$-$C_{20}$ substitué ou non, phényle, tolyle, 4-chlorophényle, phényltétrazolyle ou benzothiazolyle, Q est H ou $O_2^{\ominus}$ ; et Ar est un groupe phényle ou tolyle et

(c) un alkylaldéhyde énolisable en $C_2$-$C_{15}$ contenant un hydrogène en $\alpha$, dans un solvant organique des réactifs.

2. Procédé selon la revendication 1, dans lequel le solvant organique est un solvant organique polaire choisi parmi l'éthanol, le méthanol, l'acétonitrile et le diméthylformamide (DMF).

3. Procédé selon les revendications 1-2, dans lequel la réaction est mise en oeuvre à une température dans la gamme de 15 à 30 ° C.

4. Procédé selon les revendications 1-3, pour préparer une 4-phénylthio ou sulfonyl-1,2,3,4-tétrahydroquinoléine représentée par la formule :

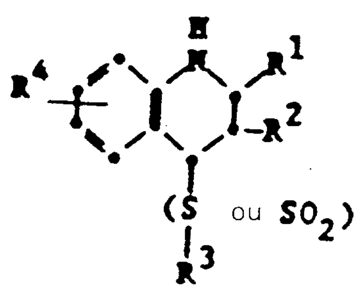

comprenant l'étape de réaction d'un sel d'aniline représenté par la formule :

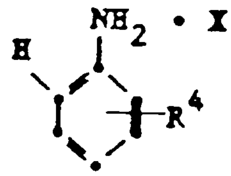

avec un thiol ou un sulfinate représenté par la formule $R^3$-SH ou $R^3$-$SO_2^\ominus$ et un alkylaldéhyde représenté par la formule $R^5$CHO dans un solvant organique pour les réactifs à une température dans la gamme de 15 à 30 ° C, dans lesquelles

$R^1$ est un groupe alkyle en $C_1$-$C_{20}$

$R^2$ est l'hydrogène ou un groupe alkyle contenant un carbone de moins que le groupe alkyle choisi pour $R^1$ ;

$R^3$ est un groupe alkyle en $C_1$-$C_{20}$ substitué ou non, phényle, tolyle, 4-chlorophényle, phényltétrazolyle ou benzothiazolyle;

$R^4$ est l'hydrogène ou un substituant qui ne gêne pas la réaction avec les autres réactifs ;

$R^5$ est un alkyle énolisable ; et

X est un sel.